(19) Europäisches Patentamt
European Patent Office
Office européen des brevets

(11) Veröffentlichungsnummer: **0 006 463**
**B1**

# (12) EUROPÄISCHE PATENTSCHRIFT

(45) Veröffentlichungstag der Patentschrift: **25.11.81**

(51) Int. Cl.[3]: **C 07 D 233/93,** C 07 D 233/94

(21) Anmeldenummer: **79101676.9**

(22) Anmeldetag: **31.05.79**

(54) **Verfahren zur Herstellung von 2-substituierten 1-Alkyl-nitroimidazolen.**

(30) Priorität: **22.06.78 DE 2827351**

(43) Veröffentlichungstag der Anmeldung: **09.01.80 Patentblatt 80/1**

(45) Bekanntmachung des Hinweises auf die Patenterteilung: **25.11.81 Patentblatt 81/47**

(84) Benannte Vertragsstaaten: **BE CH DE FR GB IT**

(56) Entgegenhaltungen:
**DE-A-1 595 928**
**DE-A-2 164 412**
**DE-A-2 521 046**
**GB-A-1 201 269**
**US-A-3 565 892**
**US-A-3 812 141**

(73) Patentinhaber: **BASF Aktiengesellschaft, Carl-Bosch-Strasse 38, D-6700 Ludwigshafen (DE)**

(72) Erfinder: **Hagen, Helmut, Dr., Max-Slevogt-Strasse 17 e, D-6710 Frankenthal (DE)**
Erfinder: **Kohler, Rolf-Dieter, Dr., Speyerer Strasse 3, D-6800 Mannheim 23 (DE)**

**EP 0 006 463 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 2-substituierten 1-Alkyl-nitroimidazolen

Die Erfindung betrifft ein Verfahren zur Herstellung von 2-substituierten 1-Alkyl-nitroimidazolen durch Umsetzung von 2-Methyl-nitroimidazolen mit Oxalsäurediestern und dann Umsetzung der gebildeten Nitroimidazolyl-(2)-brenztraubensäureester mit Chlor, Brom oder Jod und gewünschtenfalls dann durch Umsetzung der erhaltenen 2-Dihalogenmethyl-nitroimidazole mit Wasser.

Es ist aus Liebigs Ann. Chem., Band 1975, Seiten 1465 bis 1477 bekannt, daß man 2-Formyl-1-methyl-5-nitroimidazol durch Oxidation von 2-Hydroxymethyl-1-methyl-5-nitroimidazol mit Braunstein herstellen kann (loc. cit., 1466):

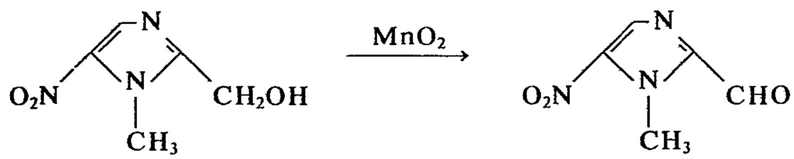


Bei Übertragung dieses Verfahrens in den technischen Maßstab treten wesentliche Schwierigkeiten auf. Man benötigt einen großen Überschuß an Braunstein. Verstopfung des Filters, Absorption von Reaktionsgemisch am Filtergut und somit auch die Verwendung großer Lösungsmittelmengen sind für das Verfahren nachteilig. Die Ausbeute an Aldehyd hängt in hohem Maße von der Aktivität des eingesetzten Braunsteins ab; der Braunstein muß deshalb stets frisch zubereitet sein. Sowohl 2-Hydroxymethyl-1-methyl-5-nitroimidazol als auch Braunstein sind teuere Ausgangsstoffe.

Ebenfalls kann man (J. Heterocycl. Chem., Band 10, Seiten 899 bis 907 (1973)) 1,2-Dimethyl-5-nitroimidazol mit N,N-Dimethylformamiddicyclohexylacetal zu 2-(2-Dimethylaminovinyl)-1-methyl-5-nitroimidazol umsetzen (loc. cit., Seite 899):

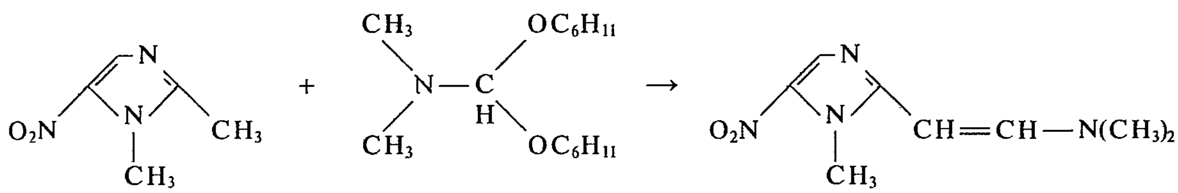

Die Oxidation der Seitenkette mit Sauerstoff in Gegenwart von Kupfer-(I)-chlorid oder Photosensibilisatoren blieb erfolglos (loc. cit., Seite 900). Erst beim Einsatz stärkerer Oxidationsmittel wie Ozon oder Osmiumtetroxid (Ann., loc. cit., Seiten 1467, 1468, 1474), gelang die Herstellung des Aldehyds in besseren Ausbeuten. Größere Mengen von 2-Formyl-1-methyl-5-nitroimidazol lassen sich jedoch auf diesem Wege nicht einfach und wirtschaftlich herstellen.

Wie die deutsche Offenlegungsschrift 2 521 046 beschreibt (Seiten 15 und 16), kann 1,2-Dimethyl-5-nitroimidazol mit Benzaldehyd in Gegenwart einer Base wie Natriumäthoxid in absolutem Äthanol zu 1-Methyl-5-nitro-2-styrylimidazol umgesetzt und dieses mit Ozon oder Alkaliperjodat und Osmiumtetroxid innerhalb von 10 bis 20 Stunden zum Aldehyd oxidiert werden.

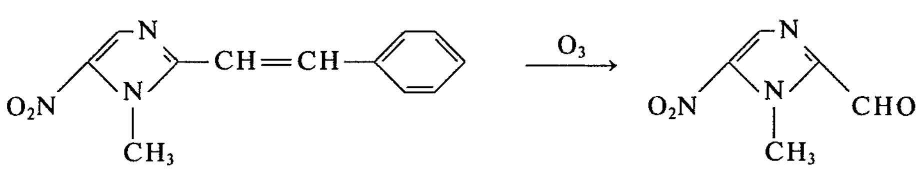


Diese Verfahren sind im Hinblick auf einfachen und wirtschaftlichen Betrieb und leicht zugängliche Reaktionskomponenten unbefriedigend.

Die Umsetzung von 1,2-Dimethyl-5-nitroimidazol mit Oxalsäureäthylesterchlorid liefert in Gegenwart von Triäthylamin während 22 Stunden unter Verwendung größerer Mengen an Äther 1-Carbäthoxy-2-(1-methyl-5-nitro-2-imidazolyl)-vinyl-äthyloxalat in 54prozentiger Ausbeute, das beim Behandeln mit Äthanol 1-Methyl-5-nitroimidazol-2-yl-brenztraubensäureäthylester in 43prozentiger Ausbeute ergibt (J. Heterocycl., loc. cit., Seiten 904 und 905)

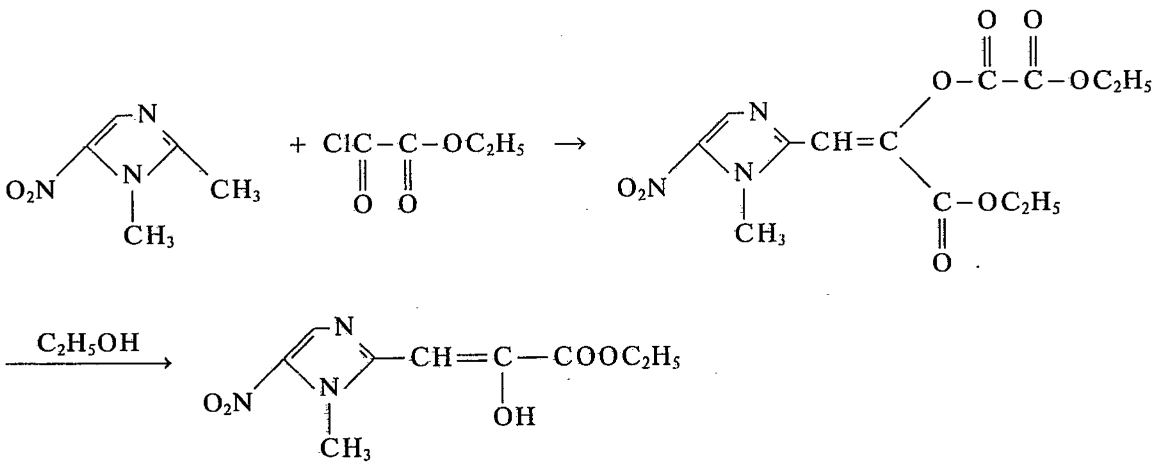


Es wurde nun gefunden, daß man 2-substituierte 1-Alkylnitroimidazole der Formel

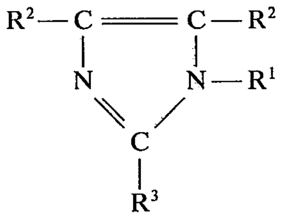

(I)

worin $R^1$ einen Alkylrest mit 1 bis 7 Kohlenstoffatomen bedeutet, ein Rest $R^2$ die Nitrogruppe und der andere Rest $R^2$ einen Alkylrest mit 1 bis 7 Kohlenstoffatomen oder ein Wasserstoffatom bezeichnet, $R^3$ für den Rest

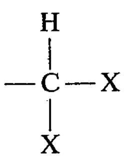

oder den Rest $-CHO$ steht und X ein Brom-, Jod- oder Chloratom bedeutet, vorteilhaft erhält, wenn man in einer ersten Stufe 2-Methyl-nitroimidazole der Formel (II)

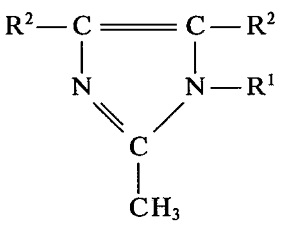

(II)

worin $R^1$ und $R^2$ die vorgenannte Bedeutung besitzen, mit Oxalsäurediestern der Formel (III)

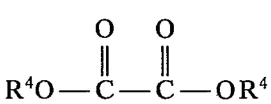

(III)

worin die einzelnen Reste $R^4$ gleich oder verschieden sein können und jeweils einen Alkylrest mit 1 bis 7 Kohlenstoffatomen, einen Cycloalkylrest mit 5 bis 6 Kohlenstoffatomen oder einen Aralkylrest mit 7 bis 12 Kohlenstoffatomen bezeichnen, in Gegenwart von Alkalialkoholaten und unter den Reaktionsbedingungen inerten, organischen Lösungsmitteln bei einer Temperatur von 0 bis 120°C umsetzt und die so erhaltenen Nitroimidazolyl-(2)-brenztraubensäureester der Formel (IV)

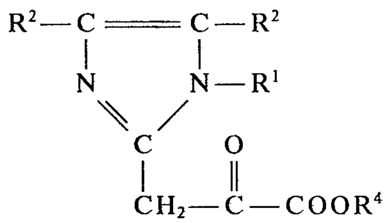

(IV)

worin $R^1$, $R^2$ und $R^4$ die vorgenannte Bedeutung besitzen, oder ihre Enolate in einer zweiten Stufe mit Brom, Jod oder Chlor in stöchiometrischer Menge oder im Überschuß in Gegenwart von Salzsäure, Schwefelsäure, Phosphorsäure, Ameisensäure, Essigsäure, Monochloressigsäure, Di- und/oder Trichloressigsäure in einer Menge von 1 bis 4 Äquivalenten Säure, oder im Falle der Verwendung von Enolaten in einer Menge von 2 bis 5 Äquivalenten Säure, bezogen auf 1 Mol Ausgangsstoff (II), und von unter den Reaktionsbedingungen organischen, inerten Lösungsmitteln und/oder Wasser bei einer Temperatur von −5 bis +70°C umsetzt und gewünschtenfalls die so erhaltenen 2-Dihalogen-methyl-nitroimidazole der Formel (V)

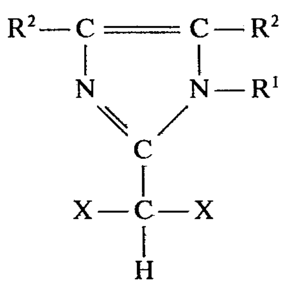

(V)

worin $R^1$, $R^2$ und X die vorgenannte Bedeutung besitzen, in einer dritten Stufe mit 150 bis 1500 Gewichtsprozent Wasser, bezogen auf Ausgangsstoff (V), bei einer Temperatur von 25 bis 150°C in Gegenwart von Salzsäure, Schwefelsäure, Phosphorsäure, Ameisensäure, Essigsäure, Monochloressigsäure, Di- und/oder Trichloressigsäure zu 2-Formyl-nitroimidazolen der Formel (VI)

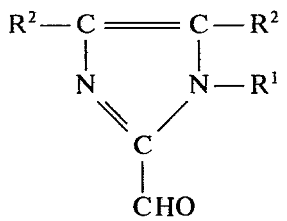

(VI)

worin $R^1$ und $R^2$ die vorgenannte Bedeutung besitzen, umsetzt, wobei die einzelnen Stufen 1, 2 und 3 entweder in einem Mehrtopfverfahren oder in einem Eintopfverfahren durchgeführt werden.

Die Umsetzung kann für den Fall der Verwendung von 1,2-Dimethyl-5-nitroimidazol, Oxalsäurediäthyläther und Chlor durch die folgenden Formeln wiedergegeben werden:

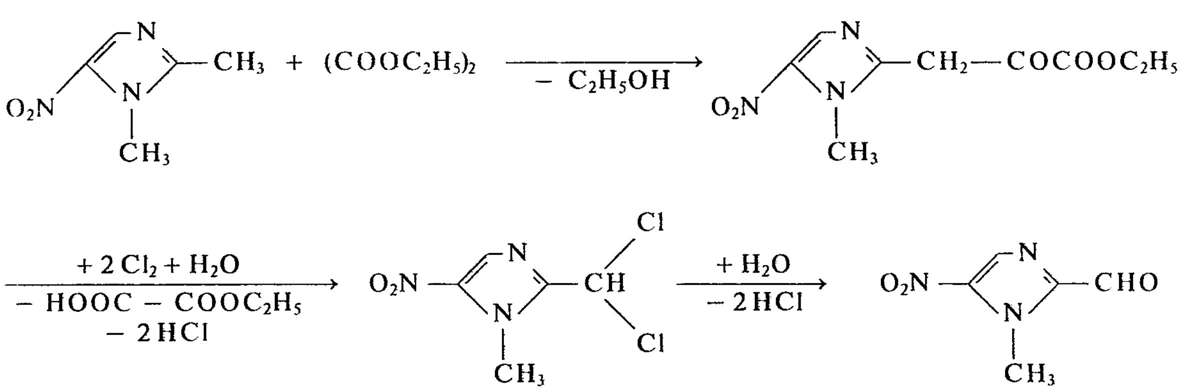


Das Verfahren nach der Erfindung liefert die neuen 2-Dihalogenmethyl-4-nitroimidazole, 2-Dihalogenmethyl-5-nitroimidazole, und 2-Formyl-4-nitroimidazole auf einfachem und wirtschaftlichem Wege und die schon beschriebenen 2-Formyl-5-nitroimidazole in besserer Ausbeute und Reinheit als die bekannten Verfahren. Oxalsäurediester sind technisch gut zugängliche Ausgangsverbindungen. Die Verwendung teurer Oxidationsmittel wie Braunstein, Edelmetalloxide

oder Ozon kann vermieden werden. Die zweite Stufe (Halogenierung) und die dritte Stufe (Hydrolyse) können auch vorteilhaft einbadig durchgeführt werden. Der gleichzeitig entstehende Oxalsäuremonoester bzw. die freiwerdende Oxalsäure kann isoliert und erneut zur Herstellung von Oxalsäurediestern eingesetzt werden. Der bei der Hydrolyse gebildete Aldehyd (I) kann ohne Isolierung für weitere Synthesen, z. B. durch Umsetzung mit Salzen des Hydroxylamins, Semicarbazids oder Thiosemicarbazids zum entsprechenden Oxim, Semicarbazon bzw. Thiosemicarbazon verwendet werden. Alle diese vorteilhaften Ergebnisse des erfindungsgemäßen Verfahrens sind überraschend, denn man hätte im Hinblick auf den Stand der Technik die Undurchführbarkeit der Halogenierung bei der Umsetzung mit Oxalsäurediestern mindestens weit schlechtere Ausbeuten und Reinheit des Stoffes (IV) und insgesamt kein gerade im industriellen Maßstab durchführbares Verfahren erwarten müssen.

Der Ausgangsstoff (II) kann mit dem Ausgangsstoff (III) in stöchiometrischer Menge oder im Überschuß, vorzugsweise in einem Verhältnis von 1 bis 4 Mol Ausgangsstoff (III) je Mol Ausgangsstoff (II), umgesetzt werden.

Folgende 2-Methyl-nitroimidazole kommen beispielsweise als Ausgangsstoffe II in Betracht: 4-Nitro-, 5-Nitro-, 4-Nitro-5-methyl-, 4-Nitro-5-äthyl-, 4-Nitro-5-propyl-, 4-Nitro-5-isopropyl-, 4-Nitro-5-butyl-, 4-Nitro-5-isobutyl-, 4-Nitro-5-sek.-butyl-, 4-Nitro-5-tert.-butyl-, 5-Nitro-4-methyl-, 5-Nitro-4-äthyl-, 5-Nitro-4-propyl-, 5-Nitro-4-isopropyl-, 5-Nitro-4-butyl-, 5-Nitro-4-isobutyl-, 5-Nitro-4-sek.-butyl-, 5-Nitro-4-tert.-butyl-1,2-dimethyl-imidazol; analog in 5-Stellung oder 4-Stellung unsubstituierte oder in 4,5-Stellung in vorgenannter Weise substituierte 1-Äthyl-, 1-Propyl-, 1-Isopropyl-, 1-Butyl-, 1-Isobutyl-, 1-sek.-Butyl-, 1-tert.-Butyl-2-methyl-imidazole.

Als Ausgangsstoffe (III) sind beispielsweise folgende Oxalsäurediester geeignet: Dimethyl-, Diäthyl-, Dipropyl-, Diisopropyl-, Dibutyl-, Diisobutyl-, Di-sek.-butyl-, Di-tert.-butyl-, Dicyclopentyl-, Dicyclohexyl-, Dibenzyl-, Diphenyl-äthyl-ester oder Monomethyl-monoäthyl-ester der Oxalsäure.

Die Umsetzung in der ersten Stufe wird in Gegenwart eines Alkalialkoholats vorteilhaft in einer Menge von 0,5 bis 5, vorzugsweise von 1 bis 3 Äquivalenten basischer Verbindung, bezogen auf ein Mol Ausgangsstoff (II), durchgeführt; unter den Alkoholaten sind Alkanolate vorteilhaft. Es kommen z. B. als Alkoholate in Frage: Natriummethylat, Natriumäthylat, Natriumpropylat, Natriumisopropylat, Natriumbutylat, Natriumisobutylat, Natrium-sek.-butylat, Natrium-tert.-butylat, Natriumäthylenglykolat, Natriumpropylen-(1,2)-glykolat, Natriumpropylen-(1,3)-glykolat, Natriumdiäthylenglykolat, Natriumtriäthylenglykolat, Natriumdipropylen-(1,2)-glykolat, Kaliummethylat, Kaliumäthylat, Kalium-n-propylat, Kaliumisopropylat, Kalium-n-butylat, Kalium-isobutylat, Kalium-sek.-butylat, Kalium-tert.-butylat, Kaliumäthylenglykolat, Kaliumpropylen-(1,2)-glykolat, Kaliumpropylen-(1,3)-glykolat, Kaliumdiäthylenglykolat, Kaliumtriäthylenglykolat, Kaliumdipropylen-(1,2)-glykolat; besonders bevorzugt sind vorgenannte Alkanolate mit 1 bis 4 Kohlenstoffatomen.

Die Umsetzung wird in der ersten Stufe bei einer Temperatur von 0 bis 120°C, vorzugsweise von 20 bis 100°C, insbesondere bei 50 bis 90°C, drucklos oder zweckmäßig unter Druck, kontinuierlich oder diskontinuierlich durchgeführt. Man verwendet unter den Reaktionsbedingungen inerte Lösungsmittel. Als Lösungsmittel kommen z. B. in Frage: aromatische Kohlenwasserstoffe, z. B. Toluol, Äthylbenzol, o-, m-, p-Xylol, Isopropylbenzol, Methylnaphthalin; Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthylhexanol, Methylglykol, n-Hexanol, Isohexylalkohol, Isoheptylalkohol, n-Heptanol, Äthylbutanol, Nonylalkohol, Dodecylalkohol, Methylcyclohexanol, Diacetonalkohol, insbesondere solche mit 1 bis 4 Kohlenstoffatomen; und entsprechende Gemische. Zweckmäßig verwendet man das Lösungsmittel in einer Menge von 100 bis 10 000 Gewichtsprozent, vorzugsweise von 200 bis 1500 Gewichtsprozent, bezogen auf Ausgangsstoff (II).

Die erste Stufe der Reaktion kann wie folgt durchgeführt werden: Ein Gemisch von Ausgangsstoff (II) und (III) zusammen mit Alkoholat und Lösungsmittel wird während 0,5 bis 24 Stunden bei der Reaktionstemperatur gehalten. Man kann den Ausgangsstoff (III) oder den Ausgangsstoff (II) zusammen mit Lösungsmittel vorlegen und dann die anderen Komponenten zugeben. Aus dem Reaktionsgemisch wird der Stoff (IV) in üblicher Weise, zweckmäßig durch Filtration, Behandeln des Filtergutes mit Säure wie wäßrige Salz- oder Schwefelsäure, und erneute Filtration, abgetrennt.

Verwendet man enolatbildende basische Verbindungen, z. B. Metallalkoholate wie Alkalialkanolat, so kann man bei der ersten Filtration im Filtergut die Enolate der Stoffe (IV) abtrennen. Die Enolate der Stoffe (IV) sind Stoffe der Formel

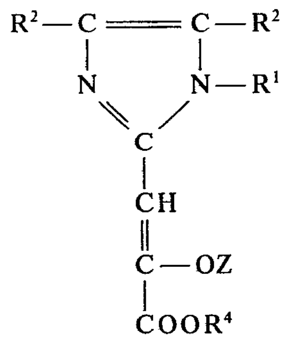

(VII)

worin $R^1$, $R^2$ und $R^4$ die vorgenannte Bedeutung besitzen und Z ein Alkalimetall bezeichnet. Bevorzugt sind Kaliumenolate und insbesondere Natriumenolate (VII). Diese Enolate (VII) können ebenfalls anstelle der Stoffe (IV) in der zweiten Reaktionsstufe umgesetzt werden.

Stoff (IV) oder Stoff (VII) wird in der zweiten Stufe des erfindungsgemäßen Verfahrens mit Brom, Jod oder insbesondere Chlor, in stöchiometrischer Menge oder im Überschuß, vorzugsweise in einem Verhältnis von 2 bis 2,5 Mol Halogen, insbesondere $Cl_2$, je Mol Ausgangsstoff II, umgesetzt. In der zweiten Verfahrensstufe wird die Umsetzung im sauren Medium durchgeführt, da ja Halogenwasserstoff gebildet wird. Man verwendet als zusätzliche Säure Salzsäure, Schwefelsäure, Phosphorsäure, Ameisensäure, Essigsäure, Monochloressersäure, Di- und/oder Trichloressigsäure in einer Menge von 1–4, vorteilhaft in einer Menge von 2 bis 2,5 Äquivalenten Säure, im Falle der Verwendung von Enolaten (VII) in einer Menge von 2–5 insbesondere von 3 bis 3,5 Äquivalenten Säure, bezogen auf 1 Mol Ausgangsstoff (II). Die Säuren können in konzentrierter Form, im Gemisch miteinander und/oder mit einem Lösungsmittel, insbesondere Wasser, angewendet werden. Bei verdünnten, wäßrigen Säuren sind 15- bis 35gewichtsprozentige Säuren, z. B. 20- bis 30gewichtsprozentige Salzsäure, 10- bis 50-, vorzugsweise 20- bis 30gewichtsprozentige Schwefelsäure oder 50- bis 100gewichtsprozentige Essigsäure, vorteilhaft. Der pH der Behandlung liegt zwischen 0 und 7, vorzugsweise zwischen 1 und 6, insbesondere zwischen 2 und 3.

Die Umsetzung der zweiten Stufe wird bei einer Temperatur von −5 bis +70°C, vorzugsweise von 0 bis 50°C, insbesondere von 10 bis 25°C, bei Unterdruck, Überdruck oder drucklos, diskontinuierlich oder kontinuierlich, durchgeführt. Man verwendet unter den Reaktionsbedingungen inerte organische Lösungsmittel und/oder Wasser. Als Lösungsmittel kommen z. B. die für die Umsetzung der ersten Verfahrensstufe genannten Lösungsmittel oder bevorzugt Wasser und entsprechende Gemische in Frage. Zweckmäßig verwendet man das Lösungsmittel in einer Menge von 100 bis 10 000 Gewichtsprozent, vorzugsweise von 200 bis 1500 Gewichtsprozent, bezogen auf Ausgangsstoff (II).

Die Reaktion kann wie folgt durchgeführt werden: Ein Gemisch von Ausgangsstoff (IV) oder (VII), Halogen, Lösungsmittel und zusätzliche Säure wird während 1 bis 24 Stunden bei der Reaktionstemperatur gehalten. Dann wird der Stoff (V) aus dem Gemisch in üblicher Weise, z. B. durch Filtration, Verdünnen des Filtrats mit Eiswasser und Neutralisation z. B. mit Alkalilauge und Filtration, abgetrennt.

Gegebenenfalls kann man die Stufen 1 und 2 des erfindungsgemäßen Verfahrens auch einbadig durchführen, indem man z. B. diue Umsetzung der Stufe 1 in vorgenannter Weise durchführt, dem Reaktionsgemisch entsprechend den vorgenannten Verfahrensbedingungen der Stufe 2 Zusatzsäure, Lösungsmittel, z. B. Wasser und Halogen zugibt und die Umsetzung der Stufe 2 durchführt.

Man kann der zweiten Stufe vorteilhaft auch die dritte Verfahrensstufe anschließen. Ausgangsstoff (V) wird mit Wasser in einer Menge von 150 bis 1500, insbesondere von 250 bis 800 Gewichtsprozent Wasser, bezogen auf den Ausgangsstoff (V), umgesetzt. Das Wasser wird zweckmäßig ganz oder teilweise in Gestalt von verdünnter Säure zugeführt. Da auch in dieser Stufe Halogenwasserstoff frei wird, findet die Umsetzung in saurem Medium statt. Man verwendet als zusätzliche Säure Salzsäure, Schwefelsäure, Phosphorsäure, Ameisensäure, Essigsäure, Monochloressigsäure, Di- und/oder Trichloressigsäure, vorteilhaft in einer Menge von 0,5 bis 1,5 Äquivalenten Säure, bezogen auf ein Mol Stoff (V). Die Säuren können in konzentrierter Form, im Gemisch miteinander und/oder mit einem Lösungsmittel, insbesondere Wasser, angewendet werden. Bei verdünnten, wäßrigen Säuren sind 3- bis 30gewichtsprozentige Säuren, z. B. bis 15gewichtsprozentige Salzsäure, von 3- bis 15-, vorzugsweise 5- bis 15gewichtsprozentige Schwefelsäure oder 5- bis 20gewichtsprozentige Phosphorsäure, vorteilhaft. Bevorzugt sind Salzsäure oder Schwefelsäure.

Die Umsetzung der dritten Stufe wird bei einer Temperatur von 25 bis 150, vorzugsweise von 80 bis 110°C, drucklos oder unter Druck, kontinuierlich oder diskontinuierlich durchgeführt. Neben Wasser sind zusätzliche, unter den Reaktionsbedingungen inerte, organische Lösungsmittel nicht notwendig, gegebenenfalls kann man die schon für die erste und zweite Verfahrensstufe genannten Lösungsmittel in den angegebenen Mengen mitverwenden.

Die Reaktion kann wie folgt durchgeführt werden: Ein Gemisch von Ausgangsstoff (V), Wasser und Säure wird während 0,5 bis 3 Stunden bei der Reaktionstemperatur gehalten. Dann wird aus dem

Reaktionsgemisch der Endstoff in üblicher Weise, z. B. durch Neutralisation, Extraktion mit einem geeigneten Lösungsmittel, wie Methylenchlorid, und Destillation des Extraktes, abgetrennt.

Man kann auch die zweite und dritte Stufe einbadig durchführen, indem man z. B. die Umsetzung der Stufe 2 in vorgenannter Weise durchführt, dem Reaktionsgemisch entsprechend den vorgenannten Verfahrensbedingungen der Stufe 3 gegebenenfalls Zusatzsäure und Wasser zugibt und die Umsetzung der Stufe 3 durchführt.

Die nach dem Verfahren der Erfindung herstellbaren neuen und bekannten Endstoffe (I) bzw. Stoffe (V) und (VI) sind wertvolle Ausgangsstoffe für die Herstellung von Farbstoffen, Schädlingsbekämpfungsmitteln und Pharmazeutika. So können sie z. B. durch Umsetzung mit 3-Amino-5-diäthylaminomethyloxazolidon-2 in die (1-Alkyl-5-nitroimidazol-2-yl)-N-(5-diäthylaminomethyl-oxazolidin-2-on-3-yl)-azomethine umgewandelt werden, die gegen Trichomonaden selektive Pharmazeutika darstellen. Bezüglich der Verwendung verweisen wir auf die genannten Veröffentlichungen.

Die in den folgenden Beispielen aufgeführten Teile bedeuten Gewichtsteile. Sie verhalten sich zu den Volumenteilen wie Kilogramm zu Liter.

## Beispiel 1

### a) 1-Methyl-5-nitroimidazol-2-yl-brenztraubensäureäthylester

Eine Mischung aus 180 Teilen 30gewichtsprozentiger Natriummethylat-Lösung (in Methanol), 146 Teilen Oxalsäurediäthylester und 150 Volumenteilen Äthanol gibt man innerhalb von 30 Minuten bei 60°C zu einer Suspension von 141 Teilen 1,2-Dimethyl-5-nitroimidazol in 250 Volumenteile Äthanol. Nach einstündigem Rühren bei 60°C wird das Gemisch abgekühlt und das Natriumenolat des 1-Methyl-5-nitroimidazol-2-yl-brenztraubensäureäthylesters abgesaugt. Das Salz wird dann unter Rühren in 1300 Volumenteile 3gewichtsprozentige Salzsäure eingetragen. Man rührt das Gemisch noch 1/2 Stunde und saugt ab. Man erhält 190 Teile (79% der Theorie) 1-Methyl-5-nitroimidazol-2-yl-brenztraubensäureäthylester vom Schmelzpunkt (Äthanol) 141°C.

### b) 2-Dichlormethyl-1-methyl-5-nitroimidazol

In eine Suspension von 241 Teilen 1-Methyl-5-nitroimidazol-2-yl-brenztraubensäureäthylester in 400 Teilen Wasser werden bei 20°C 142 Teile Chlor eingeleitet. Gleichzeitig gibt man innerhalb von 30 Minuten 400 Volumenteile 50gewichtsprozentige Schwefelsäure zu. Nach Beendigung der Reaktion (insgesamt 1,5 Stunden) kühlt man das Gemisch und saugt die ausgefallene Oxalsäure ab. Anschließend gießt man die klare Lösung in Eiswasser und neutralisiert das Gemisch unter Kühlung mit 20gewichtsprozentiger, wäßriger Natronlauge. Der ausgefallene Endstoff wird abgesaugt und getrocknet. Man erhält 157 Teile (75% der Theorie) vom Fp 52°C.

### c) 2-Formyl-1-methyl-5-nitroimidazol

210 Teile 2-Dichlormethyl-1-methyl-5-nitroimidazol werden in 800 Volumenteilen 10gewichtsprozentiger Schwefelsäure 2 Stunden bei 100°C gerührt. Unter starker Kühlung wird mit 550 Teilen 6n-Natronlauge neutralisiert und die gebildete Lösung 4mal mit je 500 Volumenteilen Methylenchlorid extrahiert. Die vereinigten Extrakte werden getrocknet, Man erhält 126 Teile (82% der Theorie) 2-Formyl-1-methyl-5-nitroimidazol vom Fp (Toluol) 93°C.

## Beispiel 2

### a) 1-Methyl-4-nitroimidazol-2-yl-brenztraubensäuremethylester

Zu einer auf 80°C erwärmten Suspension von 70,5 Teilen 1,2-Dimethyl-4-nitroimidazol in 350 Volumenteilen Toluol gibt man eine Mischung aus 73 Teilen Oxalsäurediäthylester, 250 Volumenteilen Toluol und 90 Teilen 30gewichtsprozentiges Natriummethylat (in Methanol) zu. Nach zweistündigem Rühren bei 80°C wird das Gemisch abgekühlt und abgesaugt. Man behandelt das Filtergut mit Methylenchlorid und suspendiert das gebildete Natriumenolat in Wasser. Das Gemisch wird mit 100 Teilen 18gewichtsprozentiger Salzsäure neutralisiert. Der ausgefallene 1-Methyl-4-nitroimidazol-2-yl-brenztraubensäuremethylester wird abgesaugt, getrocknet und in Methylenchlorid aufgenommen. Anschließend wird vom ungelösten Anteil abfiltriert und das Filtergut im Vakuum zur Trockne eingeengt. Man erhält 45 Teile 1-Methyl-4-nitroimidazol-2-yl-brenztraubensäuremethylester (40% der Theorie) vom Fp 186°C.

b) 2-Dichlormethyl-1-methyl-4-nitroimidazol

In eine Lösung von 11,3 Teilen 1-Methyl-4-nitroimidazol-2-yl-brenztraubensäuremethylester in 75 Volumenteilen 30gewichtsprozentiger Schwefelsäure werden bei 20°C innerhalb von 30 Minuten 8 Teile Chlor eingeleitet. Die Lösung wird dann auf Eis gegossen und unter Kühlung mit 90 Teilen 20gewichtsprozentiger Natronlauge neutralisiert. Das ausgefallene 2-Dichlormethyl-1-methyl-4-nitroimidazol wird abgesaugt. Man erhält 5 Teile (47% der Theorie) 2-Dichlormethyl-1-methyl-4-nitroimidazol vom Fp. 153°C.

c) 2-Formyl-1-methyl-4-nitroimidazol

21 Teile 2-Dichlormethyl-1-methyl-4-nitroimidazol werden in 80 Volumenteilen 10gewichtsprozentiger Schwefelsäure 2 Stunden bei 100°C gerührt. Die Lösung wird abgekühlt, auf Eis gegossen und mit 55 Teilen 6 n-Natronlauge neutralisiert. Die wäßrige Phase wird 4mal mit je 50 Volumenteilen Methylenchlorid extrahiert. Die vereinigten Extrakte werden getrocknet. Man erhält 13 Teile 2-Formyl-1-methyl-4-nitroimidazol (83% der Theorie) vom Fp. 129°C.

**Patentanspruch**

Verfahren zur Herstellung von 2-substituierten 1-Alkylnitroimidazolen der Formel (I)

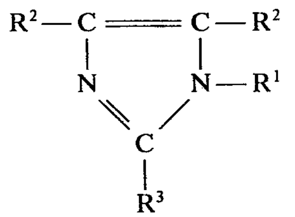

(I)

worin
$R^1$ einen Alkylrest mit 1 bis 7 Kohlenstoffatomen bedeutet,
ein Rest $R^2$ die Nitrogruppe und der andere Rest $R^2$ einen Alkylrest mit 1 bis 7 Kohlenstoffatomen oder ein Wasserstoffatom bezeichnet,
$R^3$ für den Rest

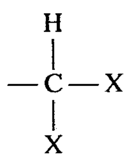

oder den Rest —CHO steht und
X ein Brom-, Jod- oder Chloratom bedeutet, dadurch gekennzeichnet, daß man in einer ersten Stufe 2-Methylnitroimidazole der Formel (II)

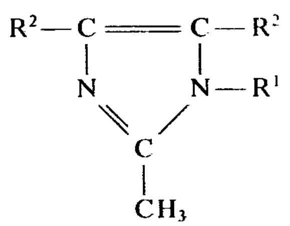

(II)

worin $R^1$ und $R^2$ die vorgenannte Bedeutung besitzen, mit Oxalsäurediestern der Formel (III)

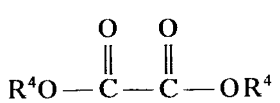

(III)

worin die einzelnen Reste $R^4$ gleich oder verschieden sein können und jeweils einen Alkylrest mit 1 bis 7 Kohlenstoffatomen, einen Cycloalkylrest mit 5 bis 6 Kohlenstoffatomen oder einen Aralkylrest mit 7 bis 12 Kohlenstoffatomen bezeichnen, in Gegenwart von Alkalialkoholaten und unter den Reaktionsbedingungen inerten, organischen Lösungsmitteln bei einer Temperatur von 0 bis 120°C

umsetzt und die so erhaltenen Nitroimidazolyl-(2)-brenztraubensäureester der Formel (IV)

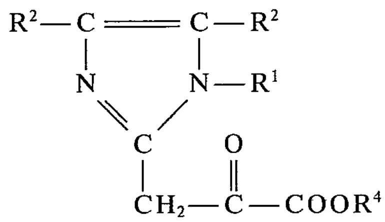

(IV)

worin $R^1$, $R^2$ und $R^4$ die vorgenannte Bedeutung besitzen, oder ihre Enolate in einer zweiten Stufe mit Brom, Jod oder Chlor, in stöchiometrischer Menge oder im Überschuß in Gegenwart von Salzsäure, Schwefelsäure, Phosphorsäure, Ameisensäure, Essigsäure, Monochloressigsäure, Di- und/oder Trichloressigsäure in einer Menge von 1 bis 4 Äquivalenten Säure, oder im Falle der Verwendung von Enolaten in einer Menge von 2 bis 5 Äquivalenten Säure, bezogen auf 1 Mol Ausgangsstoff (II), und von unter den Reaktionsbedingungen inerten, organischen Lösungsmitteln und/oder Wasser bei einer Temperatur von −5 bis +70°C umsetzt und gewünschtenfalls die so erhaltenen 2-Dihalogenmethyl-nitroimidazole der Formel (V)

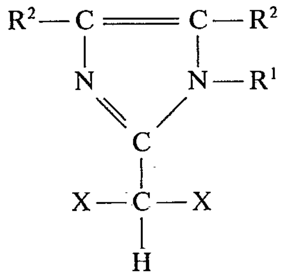

(V)

worin $R^1$, $R^2$ und X die vorgenannte Bedeutung besitzen, in einer dritten Stufe mit 150 bis 1500 Gewichtsprozent Wasser, bezogen auf Ausgangsstoff V, bei einer Temperatur von 25 bis 150°C in Gegenwart von Salzsäure, Schwefelsäure, Phosphorsäure, Ameisensäure, Essigsäure, Monochloressigsäure, Di- und/oder Trichloressigsäure zu 2-Formyl-nitroimidazolen der Formel (VII)

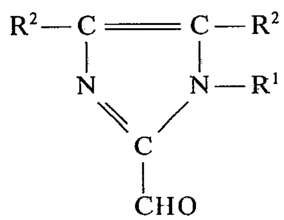

(VI)

worin $R^1$ und $R^2$ die vorgenannte Bedeutung besitzen, umsetzt, wobei die einzelnen Stufen 1, 2 und 3 entweder in einem Mehrtopfverfahren oder in einem Eintopfverfahren durchgeführt werden.

**Claim**

A process for the preparation of a 2-substituted 1-alkyl-nitroimidazole of the formula (I)

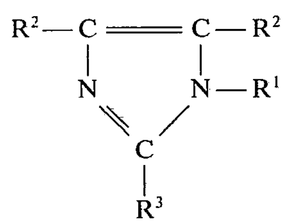

(I)

where
$R^1$ is alkyl of 1 to 7 carbon atoms,
one $R^2$ is nitro and the other $R^2$ is alkyl of 1 to 7 carbon atoms or hydrogen,
$R^3$ is

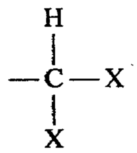

or —CHO and

X is bromine, iodine or chlorine, characterized in that, in a first stage, a 2-methylnitroimidazole of the formula (II)

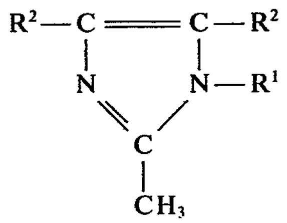

(II)

where $R^1$ and $R^2$ have the above meanings, is reacted with an oxalic acid diester of the formula (III)

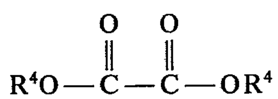

(III)

where the individual radicals $R^4$ may be identical or different and each is alkyl of 1 to 7 carbon atoms, cycloalkyl of 5 or 6 carbon atoms or aralkyl of 7 to 12 carbon atoms, in the presence of an alkali metal alcoholate and of an organic solvent which is inert under the reaction conditions, at a temperature of from 0° to 120° C, and the resulting nitroimidazol-2-yl-pyruvic acid ester of the formula (IV)

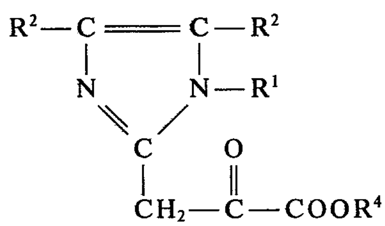

(IV)

where $R^1$, $R^2$ and $R^4$ have the above meanings, or its enolate, is reacted, in a second stage, with bromine, iodine or chlorine in the stoichiometric acmount or in excess in the presence of hydrochloric acid, sulfuric acid, phosphoric acid, formic acid, acetic acid, monochloroacetic acid, di- and/or trichloroacetic acid in an amount of 1 to 4 equivalents of acid or, when using an enolate, in an amount of 2 to 5 equivalents of acid per mole of starting material (II), and of an organic solvent which is inert under the reaction conditions and/or water, at a temperature of from −5° to +70° C, and, if desired, the resulting 2-dihalomethylnitroimidazole of the formula (V)

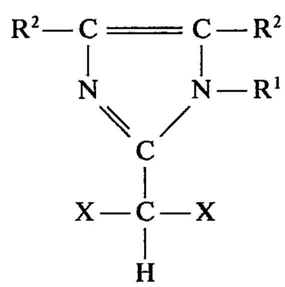

(V)

where $R^1$, $R^2$ and X have the above meanings, is reacted, in a third stage, with 150 to 1500% by weight, based on starting material (V), of water at a temperature of from 25° to 150° C in the presence of hydrochloric acid, sulfuric acid, phosphoric acid, formic acid, acetic acid, monochloroacetic acid, di- and/or trichloroacetic acid to give a 2-formylnitroimidazole of the formula (VI)

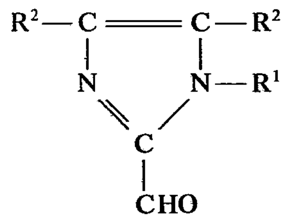

(VI)

where $R^1$ and $R^2$ have the above meanings, individual stages 1, 2 and 3 being carried out either in a multi-vessel process or in a single-vessel process.

**Revendication**

Procédé de préparation de 1-alcoyl-nitro-imidazoles substitués en position 2, de la formule (I)

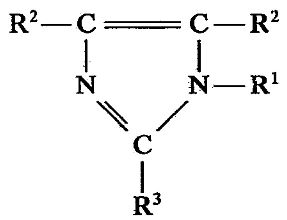

(I)

dans laquelle
$R^1$ désigne un radical alcoyle en $C_1$ à $C_7$;
l'un des substituants $R^2$ désigne un groupe nitro et l'autre un radical alcoyle en $C_1$ à $C_7$ ou un atome d'hydrogène;
$R^3$ représente un groupe

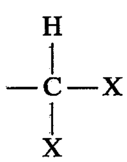

ou un groupe —CHO et
X un atome de brome, d'iode ou de chlore, caractérisé en ce que l'on fait réagir,
dans un premier stade, des 2-méthyl-nitro-imidazoles de la formule (II)

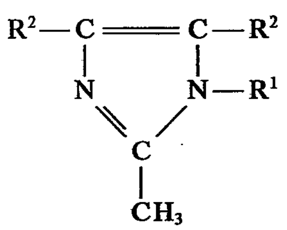

(II)

dans laquelle $R^1$ et $R^2$ possèdent les significations définies, avec des diesters de l'acide oxalique de la formule (III)

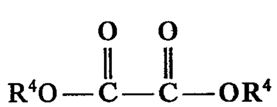

(III)

dans laquelle les substituants $R^4$, qui peuvent être identiques ou différents, désignent chacun un radical alcoyle en $C_1$ à $C_7$, un groupe cyclo-alcoyle en $C_5$ ou $C_6$ ou un groupe aralcoyle en $C_7$ à $C_{12}$,
dans des solvants organiques inertes dans les conditions réactionnelles à une température de 0 à 120° C en présence d'alcoolates de métaux alcalins, et,
dans un deuxième stade, les esters de l'acide nitro-imidazolyl-(2)-pyruvique formés, de la formule (IV)

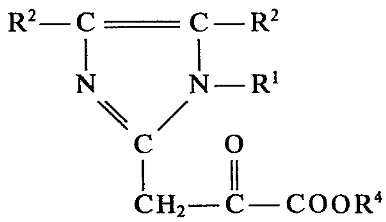

(IV)

dans laquelle R¹, R² et R⁴ possèdent les significations définies, ou leurs énolates, avec une proportion stoechiométrique ou un excès de brome, d'iode ou de chlore, en présence d'acide chlorhydrique, d'acide sulfurique, d'acide phosphorique, d'acide formique, d'acide acétique, d'acide monochlor-acétique, d'acide dichlor- et(ou) trichlor-acétique en une proportion de 1 à 4 équivalents d'acide, ou dans le cas de l'utilisation des énolates, de 2 à 5 équivalents d'acide par mole de composé de départ (II), dans des solvants organiques inertes dans les conditions réactionnelles et(ou) de l'eau, à une température de $-5$ à $+70$°C, et, le cas échéant,
on transforme les 2-dihalo-méthyl-nitro-imidazoles formés, de la formule (V)

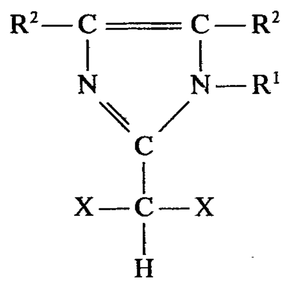

(V)

dans laquelle R¹, R² et X possèdent les significations définies,
dans un troisième stade, par réaction à une température de 25 à 150°C, en présence d'acide chlorhydrique, d'acide sulfurique, d'acide phosphorique, d'acide formique, d'acide acétique, d'acide monochlor-acétique, d'acide dichlor- et(ou) trichlor-acétique, avec 150 à 1500% en poids, par rapport au composé de départ (V), d'eau en 2-formyl-nitro-imidazoles de la formule (VI)

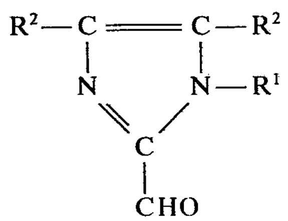

(VI)

dans laquelle R¹ et R² possèdent les significations définies, les stades 1, 2 et 3 pouvant être réalisés dans le même réacteur ou dans des réacteurs distincts.